# EUROPEAN PATENT SPECIFICATION

(11) **EP 0 730 902 B1**
(45) Date of publication and mention of the grant of the patent: **10.10.2001**
(21) Application number: 96301601.9
(22) Date of filing: 08.03.1996
(51) Int. Cl.: B01J 4/00, F25J 1/00, C01B 7/07, C07C 17/10, B01J 19/12

(54) **Chlorine gas supply to a reactor for chlorination of liquid methyl chloride**
Chlorgasversorgung eines Reaktors für die Chlorierung von flüssigem Methylchlorid
Alimentation du chlore gazeux à un reactor pour la chloration du chlorure du méthyle liquide

(30) Priority: 09.03.1995 JP 4951995
(43) Date of publication of application: 11.09.1996
(73) Proprietor: TOKUYAMA CORPORATION, Tokuyama-shi, Yamaguchi-ken 745 (JP)
(72) Inventor: Okabe, Itaru, Tokuyama-shi, Yamaguchi-ken 745 (JP)
(74) Representative: Woods, Geoffrey Corlett

(56) References cited:
- CH-A- 199 884
- DE-A- 2 346 683
- US-A- 4 230 673
- US-A- 4 321 795
- US-A- 4 614 572
- PATENT ABSTRACTS OF JAPAN vol. 12, no. 332 (M-738), 8 September 1988 & JP-A-63 096398 (KOBE STEEL LTD.), 27 April 1988,

## Description

This invention relates to a method for supplying a chlorine gas to a reactor.

Various chlorination reactions are known such as a reaction for producing methylene chloride, chloroform and carbon tetrachloride by chlorinating methyl chloride. When such chlorination reactions are carried out in a large-scale plant, chlorine gas is typically transferred from a chlorine gas supply facility into a reactor under pressure, using a reciprocating compressor or the like.

However, the introduction of chlorine gas into a reactor under pressure using a compressor puts a great strain on the compressor. The compressor is liable to break down due to the great strain on it, as a result it is difficult to provide a stable supply of chlorine gas, especially when the chlorination reaction is carried out under a high pressure.

Chlorine gas typically contains various impurities which have an adverse effect on many chlorination reactions. For instance, when a chlorine gas is transferred from a facility such as an electrolysis plant for sodium chloride, the chlorine gas generally contains of the order of several ppm of particles of a metal such as iron or the like, which is used to suppress the generation of radicals. In this case, when the chlorination reaction to be effected is a radical reaction, such iron particles lower the rate of the chlorination reaction and cause fluctuations in pressure and temperature during the reaction. Further, the chlorine gas sometimes contains several ppm of nitrogen trichloride which reacts with almost all kinds of organic compounds violently and hence, there is a risk of explosion in the reactor. Nitrogen and chlorine generated by the decomposition of nitrogen trichloride in a chlorination reaction react with each other to generate a scale of ammonium chloride. Such scale may contaminate the surface of the ultraviolet lamp and thus make a photochemical reaction unstable. Hence, it is desirable to suppress the supply of a chlorine gas containing nitrogen trichloride to a reactor for a chlorination reaction as much as possible.

US-A-4321795 describes a process for the purification of gaseous chlorine by means of compression, liquefication and re-evaporation.

It is an object of the present invention to provide a novel method for supplying a chlorine gas to a reactor in which a reaction is carried out at high pressure.

Another object of the present invention is to provide a method for stably supplying a chlorine gas to a reactor with a small load.

Still another object of the present invention is to provide a method for supplying a chlorine gas to a reactor after reducing the amount of impurities undesired for a chlorination reaction.

Other objects and advantages of the present invention will become more apparent from the following description.

According to the present invention, the above objects and advantages of the present invention can be attained by a method for supplying a chlorine gas to a reactor in which methyl chloride is reacted with chlorine in the liquid phase at high pressure which method comprises the steps of:
cooling the chlorine gas to convert it into liquefied chlorine,
pumping the liquified chlorine using a pressure of from 10 to 40 kg/cm²G to transfer it to a location near the reactor, heating the liquefication chlorine to convert it into chlorine gas, and
supplying the chlorine gas to the reactor.

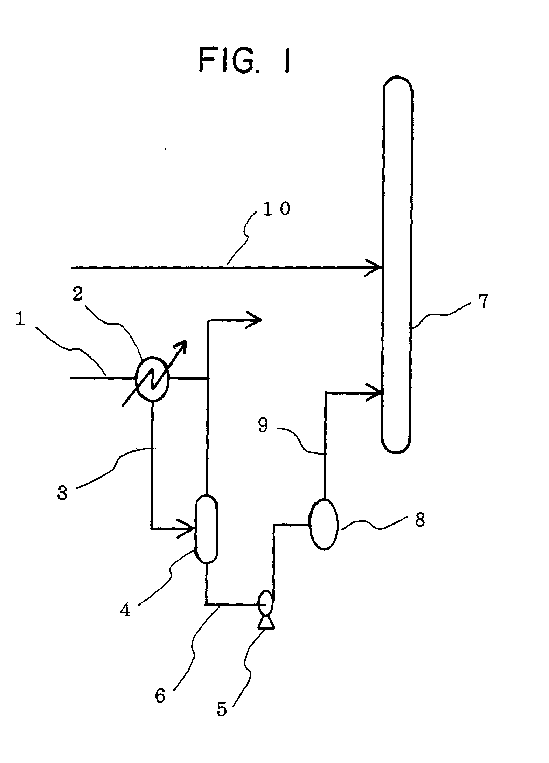

Fig. 1 is a schematic diagram showing the case where a chlorine gas supply method according to a typical embodiment of the present invention is applied in a reaction for producing higher-order chlorinated methane by chlorinating methyl chloride.

In the present invention, the chlorine gas supplied from a chlorine gas supply facility is first cooled to convert it into liquefied chlorine. Then, this liquid chlorine is transferred to a location near the reactor by pressure. Thereby it is possible to transfer chlorine to a location near the reactor with ease at an increased pressure under a smaller load, compared with the case where chlorine is supplied to the reactor in the form of a gas by a compressor. By cooling this chlorine gas, an impurity gas contained in the chlorine gas, such as oxygen or the like, which has a boiling point lower than that of the chlorine gas and exerts an adverse effect on a chlorination reaction by hindering the generation of a radical can be removed.

In this respect, known methods for cooling a chlorine gas to convert it into liquefied chlorine can be employed without restriction. Generally speaking, a method for cooling a chlorine gas to a temperature lower than the boiling point of the chlorine gas under a pressure of 1 to 15 kg/cm²G is preferred. In this case, water may be used as a refrigerant for a cooler. However, since the pressure of a chlorine gas can be reduced, a hydrocarbon fluoride-based refrigerant is preferably used to cool the chlorine to a temperature of -20 to 0°C.

Any known type of pumps can be used as a pump for transferring the liquefied chlorine to a location near the reactor by pressure, without restriction. In this case, it is general that pressure used to transfer the liquefied chlorine is 10 to 40 kg/cm²G. This liquefied chlorine is generally maintained at a temperature of -20 to 0°C.

In the present invention, the liquefied chlorine transferred to a location near the reactor is heated to re-convert it into a chlorine gas which is then supplied to the reactor by vapor pressure. In the present invention, in the step of vaporizing the liquefied chlorine, impurity metal particles such as iron can be removed. Even when nitrogen trichloride is contained, it can be thermally decomposed by heating preferably at a temperature of 80°C or higher to reduce its content.

In the present invention, heating the liquefied chlorine can be effected in a known vaporizer without restriction. Preferred examples of the vaporizer include various types of evaporators such as a multitubular type, flash evaporators and the like. Heating temperature is generally 80 to 150°C. By heating the liquefied chlorine, a chlorine gas having a pressure of 10 to 40 kg/cm²G can be obtained.

The method of the present invention can be employed in the chlorination reaction for producing higher-order chlorinated methane such as methylene chloride, chloroform and carbon tetrachloride by chlorinating methyl chloride. Known chlorination reactions can be used as the reaction for producing higher-order chlorinated methane without restriction. Particularly, a method in which methyl chloride in a liquid phase is reacted with chlorine in the presence of a radical which is generated by a radical initiator and/or ultraviolet light is preferred.

Although any chlorine gas is acceptable as the chlorine gas used in the present invention, a chlorine gas having an iron content of 1 to 20 ppm is used advantageously. A chlorine gas having a nitrogen trichloride of 0.5 to 30 ppm is used more advantageously. Generally speaking, a chlorine gas which is generated by and supplied from an electrolysis plant for sodium chloride and has the above impurity concentration is preferred.

The present invention is described in detail hereinafter with reference to the drawings.

Fig. 1 is a schematic diagram showing the case where a chlorine gas supply method according to a typical embodiment of the present invention is applied in a reaction for producing higher-order chlorinated methane such as methylene chloride, chloroform and carbon tetrachloride by chlorinating methyl chloride. In Fig. 1, a chlorine gas supplied through a pipe 1 from a chlorine gas supply facility is first supplied to a chlorine gas cooler 2 which liquefies the chlorine gas. At this occasion, impurity gases having a boiling point lower than that of the chlorine gas can be removed. The thus obtained liquefied chlorine is supplied to a liquefied chlorine storage drum 4 through a pipe 3. This liquefied chlorine is transferred by pressure efficiently through a pipe 6 under a small load by means of a pump 5 to a vaporizer 8 located near a reactor 7 for higher-order chlorinated methane. The liquefied chlorine is heated by the vaporizer 8 and converted into a chlorine gas having a high pressure which is then supplied to the reactor 7 through a pipe 9. At this time, impurity metal particles such as iron are removed in the step of vaporization by heating and impurities such as nitrogen trichloride are thermally decomposed. Meanwhile, methyl chloride is also supplied to the reactor through a pipe 10 so that the chlorine gas supplied is reacted with the methyl chloride in the reactor 7 to produce higher-order chlorinated methane.

According to the present invention, in the supply of a chlorine gas to the reactor, the chlorine gas can be stably supplied to the reactor under small load. Further, impurities such as iron and nitrogen trichloride which have an adverse effect on a chlorination reaction can be reduced, fluctuations in pressure and temperature are small, and a stable reaction can be effected without the risk of explosion and the generation of a scale. As the result, a chlorinated product of interest can be produced stably and is extremely useful.

The following examples and comparative example are given to further illustrate the present invention. However, it is understood that the present invention is not limited to these examples.

### Example 1

A higher-order chlorinated methane was produced by a production process of higher-order chlorinated methane, in which the chlorine gas supply method of the present invention shown in Fig. 1 was employed. The chlorine gas supplied through a pipe 1 was a chlorine gas which was produced by an electrolysis plant for sodium chloride, had a pressure of 2 kg/cm²G and contained 5 ppm of iron and 1 ppm of nitrogen trichloride. In a chlorine gas cooler 2, the chlorine gas was cooled and converted into liquefied chlorine using, as a refrigerant, Freon cooled to -18°C by a refrigerator. While maintaining it at a temperature of -15°C, the liquefied chlorine was transferred to a vaporizer 8 consisting of a multitubular evaporator by a pressure of 33 kg/cm²G by a pump 5. Thereafter, in this vaporizer 8, the liquefied gas was vaporized into a chlorine gas under a pressure of 30 kg/cm²G using steam heated at 130°C as a heat source. The thus obtained chlorine gas had an iron content of 0.1 ppm and a nitrogen trichloride content of 0.3 ppm. The chlorine gas had a pressure of 30 kg/cm²G and was supplied to a reactor 7 having a pressure of 27 kg/cm²G. The supply of the chlorine gas to the reactor 7 was 2,300 Nm³/hour.

Meanwhile, the supply of methyl chloride from a pipe 10 to the reactor 7 was 8.3 m²/hour. The methyl chloride and chlorine gas supplied were subjected to a liquid-phase reaction at a temperature of 100°C in the presence of a radical initiator in the reactor 7.

When the above production of higher-order chlorinated methane was carried out for one year, supply of chlorine to the reactor 7 could be stably continued. When the concentration of iron in the reactor 7 was analyzed every 10 days in the production of high-order chlorinated methane, all analytical values were 0.5 ppm or less. As the result, pressure fluctuations in the reactor 7 were 0.01 kg/cm² or less and temperature variations were 0.2°C or less throughout the year.

### Comparative Example 1

Higher-order chlorinated methane was produced for one year in the same manner as in Example 1 except that a chlorine gas was supplied directly from the pipe 1 to the reactor 7 at a pressure of 30 kg/cm²G with a reciprocating compressor. As the result, the reciprocating compressor had troubles 24 times during 1-year operation, it cost dear to repair it, and since the malfunctioning compressor had to be exchanged with a spare one each such occasion, operation became unstable temporarily.

Even when a reaction was carried out in a steady state, the chlorine gas containing 5 ppm of iron and 1 ppm of nitrogen trichloride was directly supplied to the reactor 7. Therefore, the concentration of iron in the reactor 7, which was measured every 10 days was 7 ppm. The maximum pressure variation was 0.7 kg/cm²G and the maximum temperature variation was 3.0°C, making it difficult to control the reaction sometimes.

### Example 2

The chlorine gas supplied from the pipe 1 in Example 1 was changed to a chlorine gas containing 10 ppm of iron and 2 ppm of nitrogen trichloride and having a pressure of 3 kg/cm²G. In the chlorine gas cooler 2, Freon cooled to -12°C by a refrigerator was used as a refrigerant to cool the chlorine gas and convert it into liquefied chlorine. This liquefied chlorine was transferred to the vaporizer 8 consisting of a multitubular evaporator at a pressure of 30 kg/cm²G by means of the pump 5 while it was maintained at a temperature of -10°C. Thereafter, in this vaporizer 8, the liquefied chlorine was vaporized into a chlorine gas under a pressure of 27 kg/cm²G using steam heated at 100°C as a heat source. The thus obtained chlorine gas had an iron content of 0.1 ppm and a nitrogen trichloride content of 0.7 ppm. The chlorine gas having a pressure of 27 kg/cm²G was supplied to the reactor 7 having a pressure of 24 kg/cm²G. The supply of the chlorine gas to the reactor 7 was 1,500 Nm³/hour.

Meanwhile, the supply of methyl chloride from the pipe 10 to the reactor 7 was 4.8 m³/hour. In the reactor 7, the methyl chloride and chlorine gas supplied were subjected to a liquid-phase reaction at a temperature of 100°C in the presence of a radical initiator.

When the above production of higher-order chlorinated methane was carried out for one year, supply of chlorine to the reactor 7 could be stably continued. In the production of higher-order chlorinated methane, when the concentration of ion in the reactor 7 was analyzed every 10 days, all analytical values were 0.5 ppm or less. As a result, pressure variations in the reactor 7 were 0.01 kg/cm² or less and temperature variations were 0.2°C or less throughout the year.

## Claims

1. A method for supplying a chlorine gas to a reactor in which methyl chloride in the liquid phase is reacted with chlorine at high pressure, which method comprises:
cooling a chlorine gas to convert it into liquefied chlorine,
pumping the liquified chlorine using a pressure of from 10 to 40 kg/cm²G to transfer it to a location near the reactor,
heating the liquefied chlorine to convert it into a chlorine gas, and
supplying the chlorine gas to the reactor.

2. The method of claim 1, wherein chlorine is cooled to a temperature of -20 to 0°C under a pressure of 1 to 15 kg/cm²G.

3. The method of claim 1 or 2, wherein the liquified chlorine is heated at 80 to 150°C.

4. The method of any one of the preceding claims, wherein the chlorine gas before liquefication contains 1 to 20 ppm of iron.

5. The method of any one of the preceding claims wherein the chlorine gas before liquefication contains 0.5 to 30 ppm of nitrogen trichloride.

6. The method of any one of the preceding claims, wherein the reactor is used to carry out a chlorination reaction done by a radical reaction.

7. The method of any one of the preceding claims, wherein the reactor is used to react methyl chloride in a liquid phase with chlorine in the presence of a radical generated by a radical initiator and/or ultraviolet light.

## Patentansprüche

1. Verfahren zur Zufuhr eines Chlorgases zu einem Reaktor, in dem Methylchlorid in flüssiger Phase bei hohem Druck mit Chlor umgesetzt wird, wobei das Verfahren die Schritte umfasst:
Kühlen des Chlorgases, um es in verflüssigtes Chlor umzuwandeln,
Pumpen des verflüssigten Chlors an einen Ort in der Nähe des Reaktors, wobei ein Druck von 10 bis 40 kg/cm²G zu dessen Transfer verwendet wird,
Erwärmen des verflüssigten Chlors zur Umwandlung in Chlorgas und
Zuführen des Chlorgaseszu dem Reaktor.

2. Verfahren gemäß Anspruch 1, bei dem Chlor unter einem Druck von 1 bis 15 kg/cm²G auf eine Temperatur von -20 bis 0°C gekühlt wird.

3. Verfahren gemäß Anspruch 1 oder 2, bei dem verflüssigtes Chlor auf 80 bis 150°C erhitzt wird.

4. Verfahren gemäß einem der vorhergehenden Ansprüche, bei dem das Chlorgas vor dem Verflüssigen 1 bis 20 ppm Eisen enthält.

5. Verfahren gemäß einem der vorhergehenden Ansprüche, bei dem das Chlorgas vor der Verflüssigung 0,5 bis 30 ppm Stickstofftrichlorid enthält.

6. Verfahren gemäß einem der vorhergehenden Ansprüche, bei dem der Reaktor zur Durchführung einer mittels Radikalreaktion bewirkten Chlorierungsreaktion verwendet wird.

7. Verfahren gemäß einem der vorhergehenden Ansprüche, bei dem der Reaktor zur Umsetzung von Methylchlorid in flüssiger Phase mit Chlor in Gegenwart eines durch einen Radikalinitiators und/oder ultraviolettes Licht erzeugten Radikals mit Chlor umgesetzt wird.

## Revendications

1. Procédé pour alimenter du chlore gazeux dans un réacteur dans lequel on fait réagir du chlorure de méthyle en phase liquide et du chlore à pression élevée, lequel procédé comporte les étapes consistant à :
refroidir du chlore gazeux pour le convertir en chlore liquéfié,
pomper le chlore liquéfié en utilisant une pression allant de 10 à 40 kg/cm²G pour le transférer dans un emplacement proche du réacteur,
chauffer le chlore liquéfié pour le convertir en chlore gazeux, et
alimenter le chlore gazeux dans le réacteur.

2. Procédé selon la revendication 1, dans lequel le chlore est refroidi jusqu'à une température allant de -20 à 0°C sous une pression de 1 à 15 kg/cm²G.

3. Procédé selon la revendication 1 ou 2, dans lequel le chlore liquéfié est chauffé entre 80 et 150°C.

4. Procédé selon l'une quelconque des revendications précédentes, dans lequel le chlore gazeux avant liquéfaction contient de 1 à 20 ppm de fer.

5. Procédé selon l'une quelconque des revendications précédentes, dans lequel le chlore gazeux avant liquéfaction contient de 0,5 à 30 ppm de trichlorure d'azote.

6. Procédé selon l'une quelconque des revendications précédentes, dans lequel le réacteur est utilisé pour effectuer une réaction de chloration réalisée par une réaction de radical.

7. Procédé selon l'une quelconque des revendications précédentes, dans lequel le réacteur est utilisé pour faire réagir du chlorure de méthyle en phase liquide avec du chlore en présence d'un radical généré par un initiateur de radical et/ou une lumière ultraviolette.
